Europäisches Patentamt

European Patent Office

Office européen des brevets

(11) Veröffentlichungsnummer: **0 345 732**
**A2**

## (12) EUROPÄISCHE PATENTANMELDUNG

(21) Anmeldenummer: 89110231.1

(22) Anmeldetag: 06.06.89

(51) Int. Cl.⁴ **G01N 33/68 , G01N 33/58**

(30) Priorität: 09.06.88 DE 3819707

(43) Veröffentlichungstag der Anmeldung:
**13.12.89 Patentblatt 89/50**

(84) Benannte Vertragsstaaten:
**AT BE CH DE ES FR GB GR IT LI LU NL SE**

(71) Anmelder: **Max-Planck-Gesellschaft zur Förderung der Wissenschaften e.V.**
**Bunsenstrasse 10**
**D-3400 Göttingen(DE)**

Anmelder: **BioCarb AB**
**Sölvegatan 41**
**S-223 70 Lund(SE)**

(72) Erfinder: **Timpl, Rupert, Dr,**
**Julius-Härlin-Strasse 3**
**D-8035 Gauting(DE)**
Erfinder: **Wieslander, Jörgen, Dr.**
**Östervangsvägen 20**
**S-22360 Lund(SE)**

(74) Vertreter: **Huber, Bernhard, Dipl.-Chem. et al**
**Patentanwälte H. Weickmann, Dr. K. Fincke**
**F.A. Weickmann, B. Huber Dr. H. Liska, Dr. J.**
**Prechtel Möhlstrasse 22 Postfach 860 820**
**D-8000 München 86(DE)**

(54) **Verfahren zur Bestimmung eines Antikörpertiters.**

(57) Zur Bestimmung eines Antikörpertiters in menschlichen Körperflüssigkeiten nach dem Prinzip des Immunoassays durch Inkubation mit mindestens zwei Rezeptoren $R_1$ und $R_2$, von denen $R_1$ in flüssiger Phase vorliegt und eine Markierung trägt und $R_2$ die Abtrennung der an den zu bestimmenden Antikörper gebundenen Markierung vermittelt, Trennung der gebundenen von ungebundener Markierung und Messung der Markierung in einer der beiden Phasen verwendet man als einen der beiden Rezeptoren $R_1$ oder $R_2$ ein Konjugat aus Albumin und einem Disaccharid, das aus zwei α-glycosidisch miteinander verknüpften Galactoseeinheiten besteht, wobei das Disaccharid direkt an Albumin gebunden ist und als anderen Rezeptor $R_2$ oder $R_1$ ein mit dem zu bestimmenden Antikörper oder dem Komplex aus Antikörper und Konjugat spezifisch bindefähiger Rezeptor ist.

## Verfahren zur Bestimmung eines Antikörpertiters

Die Erfindung betrifft ein Verfahren zur Bestimmung eines Antikörpertiters in menschlichen Körperflüssigkeiten nach dem Prinzip des Immunoassays durch Inkubation mit mindestens zwei Rezeptoren R₁ und R₂, von denen R₁ in flüssiger Phase vorliegt und eine Markierung trägt und R₂ die Abtrennung der an den zu bestimmenden Antikörper gebundenen Markierung vermittelt, Trennung der gebundenen von ungebundener Markierung und Messung der Markierung in einer der beiden Phasen.

Gelangt eine körperfremde Substanz in den menschlichen Kreislauf, so löst sie unter bestimmten Bedingungen die Bildung von Antikörpern aus. Sie wird dann als Antigen bezeichnet. Antikörper sind Proteinmoleküle, die der menschliche oder tierische Organismus beim Kontakt mit einem Antigen produziert. Hauptsächlich werden die Antikörper in B-Lymphozyten und Plasmazellen hergestellt. Die Spezifität der Antikörper ist verschieden. Es gibt Antikörper, die sehr spezifisch auf ein ganz spezielles Antigen gerichtet sind und Antikörper, die mit einer Klasse von Antigenen reagieren können. In den menschlichen Körperflüssigkeiten sind Antikörper sowohl als Folge der natürlichen Immunität als auch als Folge von infektiösen Erkrankungen, die durchgemacht wurden, vorhanden.

Da der Körper beim Auftreten einer Infektionskrankheit Antikörper bildet, besteht hier die Möglichkeit, frühzeitig, d.h. im Anfangsstadium, eine Infektionskrankheit diagnostizieren zu können, wenn es möglich ist, den Antikörpertiter im Blut zu bestimmen. Hierzu gibt es bereits einige Ansätze. So wurde beispielsweise gefunden, daß die bei Protozoen-Erkrankungen gebildeten Antikörper mit dem Mausbasalmembranprotein Laminin reagieren und über diese Reaktion nachgewiesen werden können. Maus-Laminin ist jedoch schwierig und nur in geringen Mengen erhältlich. Außerdem ist dieses Nachweisverfahren aufwendig. Es wäre daher wünschenswert, einfachere Nachweisverfahren zur Verfügung zu haben, um die Diagnostik von Infektionskrankheiten zu erleichtern.

Es war nun Aufgabe der Erfindung, ein Verfahren zu schaffen, mit dem der Antikörpertiter in menschlichen Körperflüssigkeiten bestimmt werden kann, wobei der Nachweis möglichst spezifisch nur Antikörper gegen Krankheitserreger erfassen sollte.

Diese Aufgabe wird gelöst durch ein Verfahren zur Bestimmung eines Antikörpertiters in menschlichen Körperflüssigkeiten nach dem Prinzip des Immunoassays durch Inkubation mit mindestens zwei Rezeptoren R₁ und R₂, von denen R₁ in flüssiger Phase vorliegt und eine Markierung trägt und R₂ die Abtrennung der an den zu bestimmenden Antikörper gebundenen Markierung vermittelt, Trennung der gebundenen von ungebundener Markierung und Messung der Markierung in einer der beiden Phasen, das dadurch gekennzeichnet ist, daß einer der beiden Rezeptoren R₁ oder R₂ ein Konjugat aus Albumin und einem Disaccharid, das aus zwei α-glycosidisch miteinander verknüpften Galactoseeinheiten besteht, enthält, wobei das Disaccharid direkt an Albumin gebunden ist und daß der andere Rezeptor R₂ oder R₁ ein mit dem zu bestimmenden Antikörper oder dem Komplex aus Antikörper und Konjugat spezifisch bindefähiger Rezeptor ist.

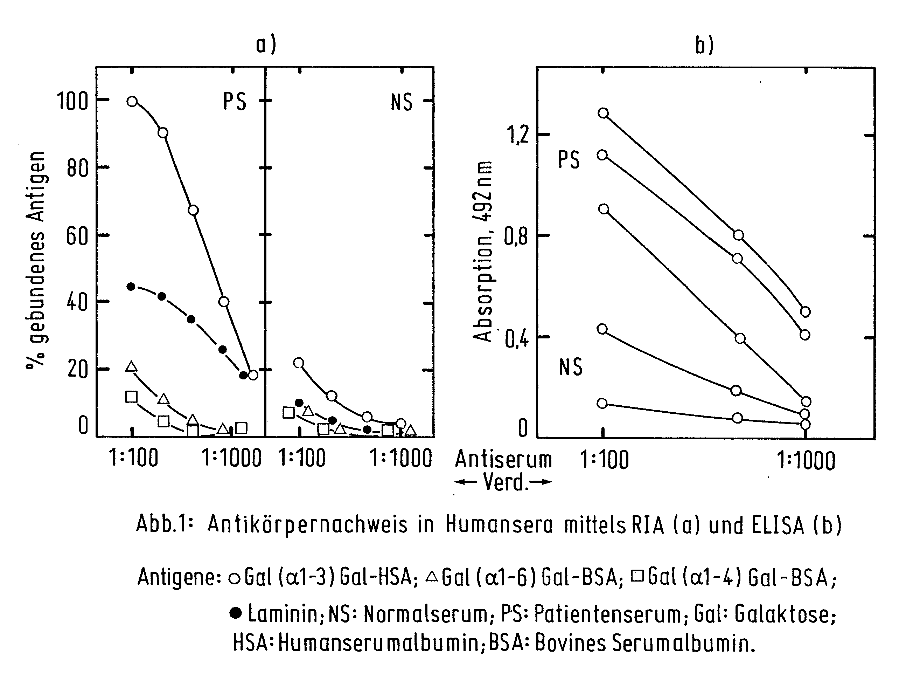

Der Antikörpertiter kann überraschenderweise in Körperflüssigkeiten mit sehr guten Ergebnissen in einfacher Weise bestimmt werden, wenn man ein Disaccharid-Albumin-Konjugat als Rezeptor verwendet. Mit diesen Konjugaten konnten Bindungswerte bis zu 100 % erhalten werden. Mit dem erfindungsgemäßen Verfahren konnten beispielsweise bei an Leishmaniasis- oder Chagas-Krankheit erkrankten Personen Antikörpertiter, die gegenüber den Normalwerten um das 10- bis 100-fache erhöht waren, festgestellt werden, wie in Fig. 1 gezeigt. Autoantikörper werden mit diesem Verfahren nicht erfaßt.

Zum Nachweis von Antikörpern nach dem Prinzip des Immunoassays sind verschiedene Verfahrensvarianten bekannt. Eine der meistverwendeten Methoden ist der sogenannte Sandwichassay. Zur Durchführung dieser Verfahrensvariante sind zwei verschiedene Rezeptoren notwendig. Der erste Rezeptor R1 liegt in flüssiger Phase vor, trägt eine Markierung und ist mit dem zu bestimmenden Antikörper bindefähig. Der zweite Rezeptor R2 ist an eine feste Phase gebunden und entweder mit dem zu bestimmenden Antikörper oder dem Komplex aus zu bestimmendem Antikörper und Rezeptor R1 bindefähig. Zur Durchführung des Bestimmungsverfahrens wird die Probelösung mit den Rezeptoren in Kontakt gebracht. Aufgrund der Bindungseigenschaften bildet sich dann an der Festphase ein Komplex aus R1, zu bestimmendem Antikörper und R2. Da der Rezeptor R1 eine Markierung trägt, kann die Menge an gebundenem Komplex nach Abtrennung der flüssigen Phase durch Auswertung der Markierung bestimmt werden. Die Menge an gebundener Markierung entspricht dann der Menge an gebundenem zu bestimmendem Antikörper. Wesentlich für die Bestimmung des Antikörpers ist daher also, einen Rezeptor zu verwenden, der spezifisch mit der gewünschten Klasse von Antikörpern reagiert.

Eine weitere Verfahrensvariante besteht darin, daß der Probelösung, die den zu bestimmenden Antikörper enthält, der erste Rezeptor $R_1$, der in flüssiger Phase vorliegt. im Überschuß zugesetzt wird. Dabei bindet der Rezeptor $R_1$ an die Paratope des zu bestimmenden Antikörpers. Anschließend wird Rezeptor $R_2$ zugegeben, der eine Präzipitation der Immunkomplexe bewirkt, wobei nicht gebundene Rezeptoren $R_1$ in der Lösung verbleiben. Nach Trennung des Präzipitates von der Flüssigkeit kann dann in einer der beiden Phasen wiederum die Markierung bestimmt werden, die ein Maß für die Menge an zu bestimmendem Antikörper ist.

Weitere, dem Fachmann bekannte Verfahrensvarianten des Immunoassays sind für das erfindungsgemäße Verfahren ebenfalls einsetzbar und brauchen hier nicht erläutert zu werden.

Erfindungsgemäß wesentlich ist die Verwendung eines Disaccharid-Konjugats, das entweder in Rezeptor $R_1$ oder Rezeptor $R_2$ enthalten ist. Das erfindungsgemäß verwendete Disaccharid-Konjugat enthält als einen Bestandteil zwei Galactose-Einheiten, die $\alpha$-glycosidisch miteinander verknüpft sind. Die Verknüpfung kann an allen zur Bindung zur Verfügung stehenden C-Atomen der Galactose erfolgen. Besonders gute Ergebnisse wurden mit Galactosyl-($\alpha$1-2)-.$\alpha$-($\alpha$1-3)-, $\alpha$-( 1-4)- und $\alpha$-( 1-6)-Galactosid erreicht. Besonders bevorzugt wird ein Konjugat eingesetzt, das $\alpha$-(1-3)-glycosidisch verknüpfte Digalactose enthält. An dieses Disaccharid wird Albumin als Trägerprotein gekuppelt. Bevorzugt wird Humanserumalbumin verwendet. Die Bindung erfolgt dabei über das reduzierende Ende des Zuckers und eine freie $NH_2$-Gruppe des Albumins. Zur Kupplung des Disaccharids an das Protein werden die beiden Komponenten in an sich bekannter Weise derivatisiert und dann miteinander umgesetzt. Das Disaccharid wird direkt ohne Verwendung eines Spacers an das Albumin gebunden.

Als bindendes Epitop dienen die beiden Galactoseeinheiten. Das Albumin hat als Trägerprotein nur die Funktion, das Molekulargewicht des Konjugats zu vergrößern und damit die Bindung zu erleichtern.

Das Disaccharid-Konjugat kann sowohl Teil des Rezeptors $R_1$ als auch des Rezeptors $R_2$ sein. Als Rezeptor $R_1$ ist an das Trägerprotein eine Markierung gebunden, damit das aus den zwei Galactoseeinheiten bestehende Epitop bindefähig bleibt. In diesem Fall ist Rezeptor $R_2$ ein mit dem zu bestimmenden Antikörper spezifisch bindefähiger Rezeptor, der in einer bevorzugten Ausführungsform eine Präzipitation des aus $R_1$ und zu bestimmendem Antikörper gebildeten Komplexes bewirkt.

Das Disaccharidkonjugat kann als Rezeptor $R_2$ an eine Festphase gebunden sein. Bei dieser Ausführungsform trägt dann der spezifische Antikörper die Markierung.

Der weiterhin für das Verfahren notwendige andere Rezeptor ist ein mit dem zu bestimmenden Antikörper oder dem Komplex aus markiertem Rezeptor bzw. festphasengebundenem und zu bestimmendem Antikörper bindefähiger Rezeptor. Er kann entweder ein spezifischer Antikörper oder ein $F(ab)_2$-Fragment sein. Bevorzugt wird hier ein gegen Humanglobulin bzw. eine Klasse von Globulinen gerichteter Antikörper oder dessen Fragment verwendet.

Der Rezeptor, der die Markierung trägt, wird als $R_1$ bezeichnet. Die Markierung kann mit einem Enzym erfolgen. Zur Bestimmung wird dann die Aktivität des Enzyms durch Umsetzen mit einem Substrat unter Bildung eines Farbstoffs in an sich bekannter Weise gemessen. Ebenso kann die Markierung eine radioaktive Substanz, eine fluoreszierende oder chemilumineszierende Substanz sein. Auch hier erfolgt die Bestimmung nach einer der dem Fachmann wohlbekannten Methoden.

Der Rezeptor $R_2$ vermittelt die Abtrennung von an den zu bestimmenden Antikörper gebundenem Rezeptor $R_1$ von in der Lösung befindlichem Rezeptor $R_1$. Je nach der Verfahrensvariante, nach der das erfindungsgemäße Verfahren durchgeführt wird, kann der Rezeptor $R_2$ ein präzipitierender Antikörper sein oder aber an eine Festphase gebunden sein. In einer bevorzugten Ausführungsform wird der Rezeptor $R_2$ an eine feste Phase gebunden. Als feste Phase geeignet sind beispielsweise Reagenzgläschen, Mikrotiterplatten oder kleine Kugeln aus Polystyrol oder ähnlichen Kunststoffen, an deren Oberfläche der Rezeptor $R_2$ adsorbiert wird.

Das erfindungsgemäße Verfahren ist auch dazu geeignet, klassenspezifische Antikörper in Körperflüssigkeiten nachzuweisen. Dazu wird in einer bevorzugten Ausführungsform des erfindungsgemäßen Verfahrens der Rezeptor $R_2$ ausgewählt nach der Immunglobulinklasse, die bestimmt werden soll. Wenn also der Titer an Immun--Globulinen bestimmt werden soll, wird als nicht disaccharidhaltiger Rezeptor ein Anti-IgG-Antikörper verwendet. Für den Nachweis von Antikörpern der Immunglobulinklassen A, E oder M werden entsprechend Anti-IgA-, Anti-IgE- oder Anti-IgM-Antikörper verwendet.

Erfindungsgemäß wird ein Verfahren zur Verfügung gestellt zur Bestimmung des Antikörpertiters in menschlichen Körperflüssigkeiten. Das Verfahren ist einfach durchzuführen und liefert sehr genaue und reproduzierbare Werte. Die eingesetzten Rezeptoren sind leicht erhältlich und gut herstellbar.

Überraschenderweise reagiert das erfindungsgemäß verwendete Disaccharid-Konjugat praktisch quantitativ mit in der Körperflüssigkeit vorhandenen Antikörpern, obwohl das Disaccharid direkt an das Protein gebunden ist ohne Zwischenschaltung eines Spacers.

Darüberhinaus ist die Bindung des erfindungsgemäß verwendeten Konjugates sehr spezifisch. So läßt sich die Bindung von 1-3-Digalactose-Konjugaten sehr effizient durch das gleiche Konjugat hemmen, nicht aber mit strukturell verschiedenen Konjugaten. Die Reaktion läßt sich nicht durch die Trägersubstanz Albumin allein hemmen. Das zeigt, daß die Bindung der Antikörper an die Konjugate über die Disaccharidgruppen und nicht über Albumin erfolgt. Daher ist jedes der verschiedenen α-Digalactose-Konjugate zum Nachweis einer unterschiedlichen Gruppe von Antikörpern mit verschiedener Spezifität geeignet.

Ein weiterer Gegenstand der Erfindung ist ein Verfahren zur Bestimmung von Digalactose-Epitopen in antikörperfreien Körperflüssigkeiten, Gewebeextrakten und oder Zellkulturmedium, das dadurch gekennzeichnet ist, daß die Probe mit einer Lösung mit bekannter Bindungskapazität für α-Di-Galactose-Epitope inkubiert und anschließend der Antikörpertiter der Lösung nach dem oben beschriebenen Verfahren bestimmt wird.

In tierischen Organismen sind Komponenten, die Galactose-Epitope besitzen, weit verbreitet. Demgegenüber sind in menschlichen Körperflüssigkeiten derartige Komponenten im Normalfall nicht nachweisbar. Bisher wurden Verbindungen, die Galactosyl-Galactosid-Epitope besitzen, in menschlichen Körperflüssigkeiten nur gefunden in Zusammenhang mit bestimmten Tumoren, z. B. Haut-, Brust-oder Lungentumoren. Derartige Komponenten in Körperflüssigkeiten nachzuweisen, ergibt daher unter Umständen eine Möglichkeit zur Diagnostik dieser Tumore. Erfindungsgemäß wird nun ein Verfahren zum Nachweis dieser Galactosyl-Galactosid-Epitope zur Verfügung gestellt, mit dem in Körperflüssigkeiten in einfacher und schneller Weise Tumorindikatoren bestimmt werden können.

Bevorzugt wird zur Bestimmung von Digalactoseepitopen als Lösung mit bekannter Bindungskapazität Serum eingesetzt, dessen Antikörpergehalt bekannt ist. Der Antikörpergehalt kann z. B. nach dem erfindungsgemäßen Verfahren bestimmt werden. Aus der Differenz der Antikörpertiterwerte vor und nach Zugabe der Lösung mit bekannter Bindekapazität kann dann die Menge an Digalactoseepitopen in der Probelösung bestimmt werden.

Die Bestimmung der Digalactoseepitope kann mit sehr hoher Empfindlichkeit durchgeführt werden. Die untere Nachweisgrenze liegt unter 0,1 ng/ml.

Ein weiterer Vorteil der erfindungsgemäßen Verfahren besteht darin, daß unter Verwendung der Digalactose-Konjugate steile lineare Eichkurven erhalten werden. Es sind daher empfindliche und quantitative Konzentrationsbestimmungen möglich.

Die Erfindung wird durch die folgenden Figuren und Beispiele erläutert.

Fig. 1A zeigt ein Diagramm, in dem die bei der Anwendung eines RIA erhaltenen Ergebnisse für Antikörpertiter in menschlichen Normal- und Patientenseren aufgetragen sind.

Fig. 1B zeigt ein Diagramm, in dem Ergebnisse, die bei Anwendung eines ELISA erhalten wurden, aufgetragen sind.

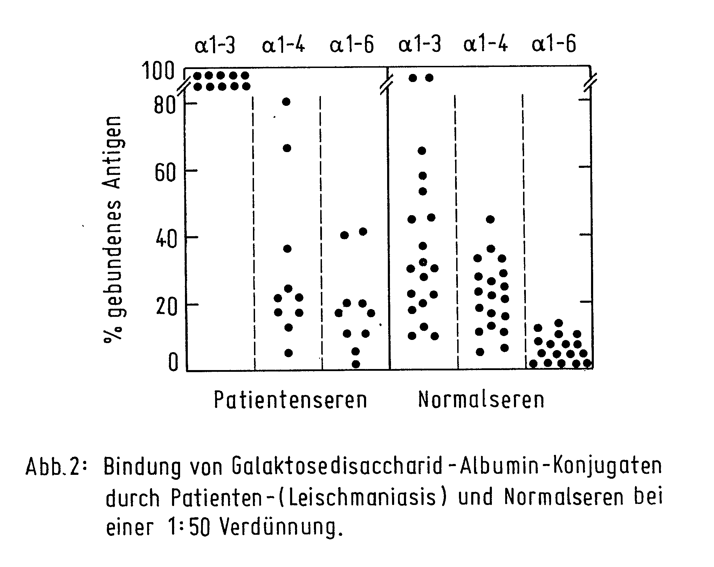

Fig. 2 zeigt ein Diagramm, in dem die Ergebnisse, die bei der Bestimmung von Antikörpertitern verschiedener Patienten erhalten wurden, aufgetragen wurden.

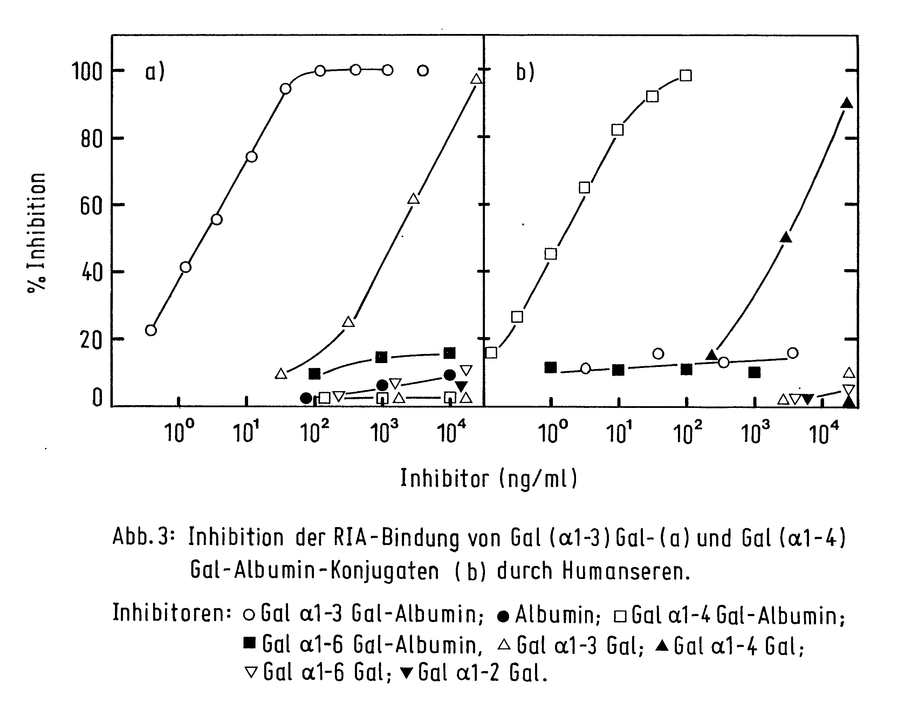

Fig. 3 zeigt ein Diagramm, in dem die Ergebnisse von Inhibitionstests aufgetragen wurden, wobei

Fig. 3A die Ergebnisse von mit RIA erhaltenen Werten darstellt, während

Fig. 3B die mit ELISA erhaltenen Werte zeigt.

**Beispiel 1**

Es wurde ein Digalactose-Albumin-Konjugat hergestellt. Dazu wurde eine Lösung von 22 mg Humanserum-Albumin in 3,5 ml Phosphatpuffer mit einem pH von 9 mit Isothiocyanat gemischt, wie es bei Zopf, D.A., Smith, D.F. Drzeniek, Z.; Tsai, C.M.; Ginsburg, V., Methods Enzymol 50 (1978), 171-175, beschrieben ist. Das erhaltene derivatisierte Humanserum-Albumin wurde dann mit p-Trifluoracetamidophenylethyl-3-O-(α-D-galactopyranosyl)-ß-D-galacto-pyranosid (0,01 mM), gelöst in 1 ml Wasser, umgesetzt. Der pH wurde auf 10 eingestellt und die Mischung übernacht gerührt. Nach Ultrafiltration und Lyophilisierung erhielt man 26 mg eines weißen Pulvers. Eine colorimetrische Bestimmung mit Anthron/Schwefelsäure ergab, daß pro Molekül Humanserum-Albumin 14 Galactosyl-Galactoside gebunden sind.

**Beispiel 2**

Es wurde ein markiertes Digalactose-Konjugat zur Verwendung in einem Radioimmunoassay hergestellt. Dazu wurden 25 μg Galactosyl-(α1-3)-Galactose-Albumin mit 0,5 mCi 125-Jod nach der Chloramin-T-Methode markiert und ungebundenes Jod durch Gelfiltration an BioGel P-2, Pharmacia, entfernt. Dieses Konjugat wurde zur Bestimmung des Antikörpertiters eingesetzt.

**Beispiel 3**

Mikrotiterplatten wurden mit einer Lösung von Galactosyl-($\alpha$1-3)-Galactose-Albumin (1 $\mu$g pro 7 ml) in 0,05 M Carbonatpuffer pH 9,8 in bekannter Weise beschichtet. Die Platten wurden dann mit unterschiedlichen Verdünnungen von Humanseren eine Stunde bei Raumtemperatur inkubiert und nicht gebundenes Protein durch Waschen mit 0,05 % Tween 20 in 0,15 M NaCl entfernt. Die Platten wurden anschließend mit Anti-Human-IgG-Antikörpern, die mit alkalischer Phosphatase konjugiert waren, inkubiert und nach erneuter Waschung wurde die Menge an gebundenem Enzym durch Reaktion mit p-Nitrophenyl-Phosphat als Substrat bestimmt.

**Beispiel 4**

Die Konzentration von Galactosyl-($\alpha$1-3)-Galactoseepitop in einer Probe wurde durch einen Inhibitionstest bestimmt. Dazu wurde ein Normalserum, dessen Antikörpergehalt vorher bestimmt worden war, mit der Probelösung 16 Stunden bei 4°C vorinkubiert und nach Zugabe von 1 ng radioaktiv markiertem Galactosedisaccharid-Albumin-Konjugat weitere 8 Stunden bei 4°C inkubiert. Anschließend wurde ein Überschuß von Antikörpern gegen Human-Immunglobulin-G zugesetzt und weitere 16 Stunden bei 4°C inkubiert. Der durch die Präzipitation ausgefällte Immunkomplex wurde durch Zentrifugation von der Lösung abgetrennt. Anschließend wurde der Anteil an gebundener Markierung bestimmt. Der erhaltene Wert wurde verglichen mit Werten, die bei Durchführung desselben Tests erhalten wurden, wobei statt der Probelösung eine Standardkonzentration von Galactose-Disaccharid-Albuminkonjugat enthalten war.

**Ansprüche**

1. Verfahren zur Bestimmung eines Antikörpertiters in menschlichen Körperflüssigkeiten nach dem Prinzip des Immunoassays durch Inkubation mit mindestens zwei Rezeptoren $R_1$ und $R_2$, von denen $R_1$ in flüssiger Phase vorliegt und eine Markierung trägt und $R_2$ die Abtrennung der an den zu bestimmenden Antikörper gebundenen Markierung vermittelt, Trennung der gebundenen von ungebundener Markierung und Messung der Markierung in einer der beiden Phasen, **dadurch gekennzeichnet,** daß einer der beiden Rezeptoren $R_1$ oder $R_2$ ein Konjugat aus Albumin und einem Disaccharid, das aus zwei $\alpha$-glycosidisch miteinander verknüpften Galactoseeinheiten besteht, enthält, wobei das Disaccharid direkt an Albumin gebunden ist und daß der andere Rezeptor $R_2$ oder $R_1$ ein mit dem

zu bestimmenden Antikörper oder dem Komplex aus Antikörper und Konjugat spezifisch bindefähiger Rezeptor ist.

2. Verfahren nach Anspruch 1, **dadurch gekennzeichnet,** daß als Disaccharid ein Galactosyl-($\alpha$ 1-2,$\alpha$-1-3, $\alpha$-1-4 und/oder $\alpha$-1-6)-Galactosid verwendet wird.

3. Verfahren nach Anspruch 1, **dadurch gekennzeichnet,** daß das Konjugat als Disaccharid ein Galactosyl-($\alpha$1-3)-Galactosid enthält.

4. Verfahren nach einem der vorhergehenden Ansprüche, **dadurch gekennzeichnet,** daß das Konjugat als Albumin Humanserumalbumin enthält.

5. Verfahren nach einem der vorhergehenden Ansprüche, **dadurch gekennzeichnet,** daß als Markierung ein Enzym, eine fluoreszierende, chemilumineszierende oder radioaktive Substanz verwendet wird.

6. Verfahren nach einem der vorhergehenden Ansprüche, **dadurch gekennzeichnet,** daß der andere Rezeptor ein Anti-Immunglobulin-Antikörper ist.

7. Verfahren nach einem der vorhergehenden Ansprüche, **dadurch gekennzeichnet,** daß der Rezeptor $R_2$ an eine Festphase gebunden ist und das Disaccharid enthält.

8. Verfahren zur quantitativen Bestimmung von $\alpha$-Digalactoseepitopen in antikörperfreien Körperflüssigkeiten, Gewebeextrakten und/oder Zellkulturmedien, **dadurch gekennzeichnet,** daß man eine Probelösung mit einer Lösung mit bekannter Bindungskapazität für $\alpha$-Digalactoseepitope inkubiert und anschließend den Antikörpertiter der Lösung mit einem Verfahren gemäß einem der Ansprüche 1 bis 7 bestimmt.

9. Verfahren nach Anspruch 8, **dadurch gekennzeichnet,** daß man als Lösung mit bekannter Bindungskapazität Humanserum verwendet, dessen Antikörpertiter bekannt ist.

10. Anwendung des Verfahrens nach einem der Ansprüche 1 bis 7 zur Bestimmung von klassenspezifischen Antikörpern in menschlichen Körperflüssigkeiten, **dadurch gekennzeichnet,** daß man als nicht Disaccharidhaltigen Rezeptor einen gegen eine Immunglobulinklasse gerichteten Antikörper verwendet.

Abb.1: Antikörpernachweis in Humansera mittels RIA (a) und ELISA (b)

Antigene: ○Gal (α1-3) Gal-HSA; △Gal (α1-6) Gal-BSA; □Gal (α1-4) Gal-BSA;
●Laminin; NS: Normalserum; PS: Patientenserum; Gal: Galaktose;
HSA: Humanserumalbumin; BSA: Bovines Serumalbumin.

Abb. 2: Bindung von Galaktosedisaccharid-Albumin-Konjugaten durch Patienten-(Leischmaniasis) und Normalseren bei einer 1:50 Verdünnung.

EP 0 345 732 A2

Abb. 3: Inhibition der RIA-Bindung von Gal (α1-3) Gal- (a) und Gal (α1-4) Gal-Albumin-Konjugaten (b) durch Humanseren.

Inhibitoren: ○ Gal α1-3 Gal-Albumin; ● Albumin; □ Gal α1-4 Gal-Albumin; ■ Gal α1-6 Gal-Albumin, △ Gal α1-3 Gal; ▲ Gal α1-4 Gal; ▽ Gal α1-6 Gal; ▼ Gal α1-2 Gal.

EP 0 345 732 A2